(19) Europäisches Patentamt
European Patent Office
Office européen des brevets

(11) Publication number: 0 304 143
B1

(12) **EUROPEAN PATENT SPECIFICATION**

(45) Date of publication of patent specification: **16.01.91**

(51) Int. Cl.$^5$: **C 02 F 3/10**, C 12 N 11/08, C 08 F 20/60, C 08 F 20/34, C 08 L 33/14

(21) Application number: **88303631.1**

(22) Date of filing: **21.04.88**

(54) **Water-absorptive resin for microbial carrier.**

(30) Priority: **19.08.87 JP 204246/87**

(43) Date of publication of application:
**22.02.89 Bulletin 89/08**

(45) Publication of the grant of the patent:
**16.01.91 Bulletin 91/03**

(84) Designated Contracting States:
**DE ES FR GB NL**

(56) References cited:
**EP-A-0 209 790**
**WO-A-81/00575**
**US-A-3 926 756**

**BIOTECHNOLOGY AND BIOENGINEERING, vol. 23, no. 11, November 1981, pages 2561-2567, John Wiley & Sons, Inc., New York, EIZO SADA ET AL:. Performance of fluidized-bed reactors utilizing magnetic fields" page 2561, line 1 - page 2563, line 11**

(73) Proprietor: **KYORITSU YUKI CO. LTD.**
**13-15 Ginza 7-chome**
**Chuo-ku Tokyo (JP)**

(72) Inventor: **Fukushima Reizou**
**8-38, 2-chome, Shimomachiya**
**Chigasaki-shi, Kanagawa-ken (JP)**
Inventor: **Aoyama Kiyoshi**
**225-3, Omagari, Samukawa-machi**
**Kouza-gun, Kanagawa-ken (JP)**

(74) Representative: **Pendlebury, Anthony et al**
**PAGE, WHITE & FARRER 54 Doughty Street**
**London WC1N 2LS (GB)**

**EP 0 304 143 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a water-absorptive resin available as a microbial carrier in a fluidized bed biodisposer for treating, for example, organic waste water.

There has been known a process wherein microorganisms are incorporated into a polymer gel carrier to thereby prevent the effusion thereof from a system. Examples of conventional polymer gels employed in these bioreactors include polyacrylamide, carrageenan and agarose $[(C_{12}H_{14}O_5(OH)_4]_n)$.

There has been further known a process wherein a microorganism is incorporated into an agarose gel containing a magnetic material for use in a microbial reaction such as methane fermentation (cf. Japanese Patent Laid-Open No. 257180/1986).

However such a bioreactor is disadvantageous because of the low efficiency caused by the slow migration of nutrients and waste matters of the incorporated microorganism in the polymer gel. In addition, the incorporation of the microorganism into the gel requires a complicated procedure. Further this bioreactor should be prepared immediately before the use since it can not withstand prolonged storage. Furthermore the microorganism, being fixed in the gel can not be renewed and thus dead cells remain in the carrier, which might inhibit the fixation of the microorganism upon prolonged operation to thereby lower the efficiency of the bioreactor. Accordingly there is a problem to be solved relating to a fluidized bed biodisposer, namely, how to efficiently stir the carrier and how to efficiently recover the carrier and replace the same with a fresh one at appropriate intervals.

It is an object of the present invention to solve the problem described above by using an acrylic cationic resin, which has a high water-absorptivity and contains a magnetic material, in a state of absorbing water as a microbial carrier for fixing a microorganism in a fluidized bed biodisposer.

It is another object of the present invention to provide a water-absorptive resin for a microbial carrier. When the water-absorptive resin as described above is introduced into a waste water as a carrier in a fluidized bed biodisposer, it would absorb a large amount of water to thereby give a cationic polymer gel comprising the water-absorbing resin containing a magnetic material. A microorganism is tightly fixed on the surface of the gel, i.e., the carrier and grows thereon. Since the microorganism layer thus formed is in direct contact with the organic waste water which is a nutritional source therefor, the microorganism can rapidly absorb nutrients and excrete waste matters.

It is still another object of the present invention to provide a water-absorptive resin for a microbial carrier in a fluidized bed biodisposer wherein a microorganism in waste water are successively renewed and thus a highly active microorganism is continuously fixed on the surface of a water-absorbing resin, i.e., the carrier.

It is still another object of the present invention to provide a water-absorptive resin for a microbial carrier in a fluidized bed biodisposer wherein the water-absorptive resin gel absorbing a large amount of waste water has nearly the same specific gravity as that of the waste water, which makes it possible to readily maintain the water-absorbing resin carrier in the waste water in a fluidized state by slightly stirring the same in the waste water with, for example, the air from a diffuser or methane gas.

It is still another object of the present invention to provide a water-absorptive resin for a microbial carrier in a fluidized bed biodisposer wherein said water-absorptive resin in a state of absorbing water, i.e., the carrier contains a magnetic material and thus can be readily recovered from the treated waste water by using a magnet.

It is still another object of the present invention to provide a water-absorptive resin for a microbial carrier in a fluidized bed biodisposer characterized in that said water-absorptive resin would exert a remarkable effect as a microbial carrier when merely introduced into water containing a microorganism, that seeding can be readily carried out, that said carrier containing a magnetic material can be readily recovered, and that the polymer gel can be fluidized by slightly stirring through, for example, air diffusion since the water-absorptive resin, i.e., the polymer gel has nearly the same specific gravity as that of the water to be treated.

It is still another object of the present invention to provide a water-absorptive resin for a microbial carrier in a fluidized bed biodisposer which can stably carry a high load compared with a conventional activated sludge process or an anaerobic methane fermentation process utilizing no distinct carrier.

In the present invention, an acrylic cationic resin of a high water-absorptivity is used as a microbial carrier in a fluidized bed biodisposer.

According to the present invention there is provided a water-absorptive resin for a microbial carrier in a fluidized bed biodisposer wherein a magnetic material is incorporated in a cationic crosslinked polymer comprising at least 10% by weight of of a cationic monomer unit(s) of the following formula:

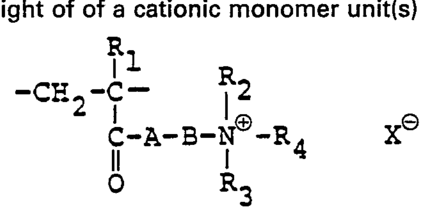

(I)

wherein A represents an oxygen atom or an NH group,

B represents a $C_2H_4$, $C_3H_6$ or $CH_2CH(OH)CH_2$ group,
$R_1$ represents a hydrogen atom or a methyl group,
$R_2$ and $R_3$ each independently represent a methyl or ethyl group,
$R_4$ represents a hydrogen atom or a methyl, ethyl, benzyl or 3-chloro-2-hydroxypropyl group, and
$X^-$ represents an anion.

When introduced into a fluidized bed biodisposer, the water-absorptive resin of the present invention would absorb the water to be treated. Thus the cationic crosslinked polymer would absorb water 10 times by weight, on a dry basis, as much as the polymer per se to thereby give a cationic polymer gel containing a magnetic material. Then a microorganism is tightly fixed onto the surface of the polymer gel and grows thereon.

A crosslinked polymer comprising at least 10% by weight of acrylic water-soluble monomer unit(s) is available as the water-absorptive resin for the microbial carrier of a fluidized bed biodisposer of the present invention, namely, as the cationic resin of a high water-absorptivity available in achieving the abovementioned objects. Such a crosslinked polymer may be prepared by copolymerizing said monomer(s) with a divinyl compound or by reacting a water-soluble cationic polymer with a polyfunctional compound to thereby form crosslinkages. Examples of the acrylic water-soluble cationic monomer available in the preparation of the polymer gel include tertiary amine salts and/or quaternary ammonium salts of dialkylaminoalkyl (meth)acrylate and dialkylaminoalkyl(meth)acrylamide. A tertiary amine may be quarternarized by using dimethyl sulfate, diethyl sulfate, methyl chloride, benzyl chloride or epichlorohydrin. Either a homopolymer of one of these cationic monomers or a copolymer thereof may be employed. In addition, a water-soluble nonionic monomer such as (meth)acrylamide may be copolymerized therewith at a ratio of 90% by weight or less based on the total monomers.

The polymer obtained by polymerizing these acrylic cationic monomers has a high molecular weight and thus gives a resin of a high gel strength, in spite of the high water-absorptivity.

It is preferable that the water-absorptive resin of the present invention be capable to absorb pure water 10 to 500 times by weight as much as itself. Generally speaking, a resin having too high a water-absorptivity would have a low gel strength, while a resin having too low a water-absorptivity would cause the local concentration of a metabolite. In contrast to these resins, when the water-absorptive resin of the present invention is used as a carrier and absorbs water, the resulting cationic polymer gel has a low degree of crosslinking and thus various nutrients and waste matters can readily migrate therein.

0.01 to 1% by weight, based on the total monomers, of a crosslinking agent is required in order to achieve an appropriate water-absorptivity of the water-absorptive resin. The crosslinkage can be formed by copolymerizing the monomer(s) with a divinyl compound, post-crosslinking the same or combining these methods.

Examples of the divinyl compound to be copolymerized include N,N-methylenebisacrylamide and N-allylacrylamide.

Examples of the polyfunctional compound to be used in post-crosslinking include not only those capable of reacting with an amine, such as epichlorohydrin, diglycidylamine and diglycidyl ether, but also aldehydes capable of reacting with copolymerized acrylamide, such as formaldehyde.

When a divinyl compound is to be copolymerized with monomer(s), the whole monomers are dissolved in water and a water-soluble free-radical initiator is added to the resulting aqueous solution to thereby give a cationic water-absorptive resin. Spherical particles highly suitable as a carrier of a fluidized bed can be obtained by pearl polymerization, wherein an aqueous solution of monomers is dispersed in an oil and polymerized therein. The crosslinking density of the surface of the resin can be elevated by adding a polyfunctional compound such as diglycidyl ether to the oil. Post-crosslinking may be similarly carried out in the case where no divinyl compound is copolymerized. Alternately the post-crosslinking may be carried out by reacting the cationic polymer in a state of an aqueous solution with a polyfunctional compound.

Examples of the magnetic material to be incorporated in the cationic resin of a high water-absorptivity include particles such as beads, powders and granules of various ferrites and iron alloys. A magnetic material of a poor corrosion resistance, for example, an iron alloy may be rendered rustproof by, for example, plating or treatment with silane ($Si_n H_{2n+2}$).

It is preferable that the magnetic material be employed at a ratio by weight of 1:10 to 10:1, on a dry basis, based on the crosslinked polymer resin to be used in the present invention. When the ratio of the magnetic material to the resin exceeds the above range, the water-absorbing resin, i.e., the gel in the biodisposer can not be satisfactorily fluidized. On the other hand, the use of an excessively small amount of the magnetic material would make the recovery of the carrier difficult. It is further preferable that the carrier in a state of absorbing water should have a particle size of 0.5 to 20 mm.

The abovementioned gel, i.e., the water-absorbing resin, is generally fluidized by flowing air thereinto through a diffuser tube in the case of an aerobic fluidized bed, or by blowing methane gas thereinto in the case of an anaerobic one. It is also possible to fluidize the same by mechanical stirring. The water-absorbing resin in the form of a carrier on which a microorganism is fixed can be readily separated from the treated water by using a magnet and returned to the biodisposer.

Since the polymer gel to be used in the present invention for fixing a microorganism comprises cationic monomer(s) as the main constituting unit(s), the surface of the water-absorbing carrier, i.e., the gel

is positively charged. Thus a microorganism, which is negatively charged in general, is adsorbed on the surface of the cationic gel to thereby form a dense microbial layer.

Nutrients for a microorganism, such as phosphate ion, which are anionic, would be concentrated within the gel and contribute to the growth of the microorganism.

Since the density of the crosslinking of this gel is low, various nutrients and waste matters can readily migrate therein.

The surface of the gel is continuously coated with a highly active microorganism, since the microbial layer adhering to the surface of the gel is successively renewed. After the completion of the reaction, the magnetic gel can be readily recovered by using a magnet and returned to the biodisposer. Although the carrier can be readily separated from the treated water, the fluidization of the gel can be achieved by slightly stirring the same.

It is described in Japanese Patent Laid-Open No. 43203/1981 that a quaternary ammonium salt-type crosslinked polymer exerts a bactericidal effect. However, the water-absorptive resin of the present invention would never exert such an effect. The following Examples are given by way of further illustration of the present invention.

## Example

(I) Synthesis Example 1

200 g of cyclohexane was introduced into a five-necked separable flask (500 ml) provided with a stirrer, a thermometer, a reflux condenser and a nitrogen inlet. 1 g of ethylcellulose (an ethyl derivative of cellulose) was added thereto and dissolved therein by heating the mixture to 60°C. Then nitrogen gas was blown into the flask to thereby replace oxygen with the same.

6 g of a triiron tetraoxide powder was dispersed in 75 g of an 80% aqueous solution of methacryloyloxyethyldimethylbenzylammonium chloride. 6.0 cc of a 10% aqueous solution of N,N-methylenebisacrylamide and 1.2 g of a 10% aqueous solution of 2,2-azobis-(2-amidinopropane) hyrochloride were aded thereto and the resulting mixture was introduced into a dropping funnel. Then nitrogen gas was passed therethrough to thereby replace oxygen with the same. The mixture was slowly added dropwise to the cyclohexane under stirring to thereby effect polymerization.

After carrying out the polymerization at 60°C for three hours, the reflux condenser was replaced with an azeotropic dehydrator and azeotropic dehydration was carried out within the flask in a water bath at 80 to 90°C under stirring. After thoroughly dehydrating, the polymer particles were filtered and the cyclohexane was removed by drying. Thus a highly water-absorptive resin was obtained in the form of beads.

These beads were screened to thereby select those having a particle size of 0.4 to 0.5 mm and the selected ones were allowed to absorb distilled water. Thus the crosslinked polymer absorbed water 35 times by weight as much as the polymer per se on a dry basis. This gel was referred to as G-1.

(II) Synthesis Example 2

125 g of an 80% aqueous solution of acryloyloxyethyltrimethylammonium chloride was introduced into a lidded glass container (300 ml) provided with a nitrogen inlet. 0.05 g of N,N-methylenebisacrylamide was dissolved therein and 100 g of triiron tetraoxide was dispersed therein. The resulting dispersion was heated to 60°C and purged with nitrogen. Then 3 ml of a 10% aqueous solution of 2,2′-azobis(2-amidinopropane) hydrochloride was added thereto and the obtained mixture was maintained at 60°C for five hours to thereby continue the polymerization. After the completion of the polymerization, the obtained mass was taken out, cut into a sheet of 5 mm in thickness and dried in a ventilated dryer at 100°C. The material thus dried was ground with a mill.

Then the ground material was screened to thereby select resin particles of 0.4 to 0.5 mm in particle size. The selected particles were allowed to absorb water. Thus this resin absorbed water 270 times by weight as much as the resin per se on a dry basis. This gel was referred to as G-2.

(III) Synthesis Example 3

10 g of a commercially available reagent-grade iron powder was introduced into a four-necked flask (300 ml) provided with a reflux condenser, a nitrogen inlet and a stirrer. 100 ml of xylene and 0.3 g of acryloyloxypropyltrimethoxysilane were added thereto and the resulting mixture was allowed to react at 60°C for 18 hours. Then 30 g of hydroxyethyl acrylate was added thereto and the mixture was purged with nitrogen. 0.1 g of azobisisobutyronitrile was further added thereto and the polymerization was continued at 60°C for six hours. The solid matters were filtered, washed and dried. The solid product thus obtained was ground and dispersed in 75 g of an 80% solution of a reaction product between N,N-dimethylaminopropylacrylamide and methyl chloride in which 0.02 g of methylenebisacrylamide was dissolved. Subsequently 1.8 ml of a 10% aqueous solution of 2,2′-azobis-(2-amidinopropane) hydrochloride was added thereto, similar to the above Synthesis Example 1, and polymerization was effected at 60°C for five hours. The obtained product was dried and ground to thereby give a magnetic, water-absorptive resin. This resin, which had a water-absorption capacity of 90-fold, was referred to as G-3.

(IV) Synthesis Example 4

2 g of N,N-dimethylaminopropylacrylamide and 18 g of acrylamide were introduced into a lidded glass container (300 ml) provided with a nitrogen inlet. These compounds were then dissolved in 180 ml of

deionized water and the pH value of the solution was adjusted to 4.5 with sulfuric acid. After subjecting the solution to nitrogen purging, 0.2 ml of a 1% aqueous solution of ammonium persulfate and 0.2 ml of a 1% aqueous solution of sodium hydrogen sulfite were added thereto to thereby effect polymerization. Five hours thereafter, 20 g of a triiron tetraoxide powder and 20 ml of formalin diluted 100-fold were added to the polymer solution and thoroughly mixed therewith. Then the mixture was transferred into a Petri dish of 15 cm in diameter and dried in a ventilated drier at 110°C. The dried matter was ground with a mill and screened to thereby select resin particles having a particle size of 0.4 to 0.5 mm. The selected particles were allowed to absorb distilled water. Thus the crosslinked polymer absorbed water 30 times by weight as much as the polymer per se on a dry basis. This gel was referred to as G-4.

(I) Comparative Synthesis Example 1

300 g of a 30% aqueous solution of acrylamide was introduced into a lidded glass container (500 ml) provided with a nitrogen inlet. 0.05 g of N,N-methylenebisacrylamide was dissolved therein and 90 g of triiron tetraoxide was dispersed therein. The resulting dispersion was subjected to nitrogen purging and 1 ml of a 1% aqueous solution of ammonium persulfate and 1 ml of a 1% aqueous solution of sodium hydrogen sulfite were added thereto at 20°C to thereby effect polymerization. After the completion of the polymerization, the obtained mass was taken out, cut into a sheet of 5 mm in thickness, dried in a ventilated drier at 100°C and ground with a mill. Then the material was screened to thereby select resin particles having a particle size of 0.4 to 0.5 mm. The resin thus selected was allowed to absorb distilled water. As a result, this crosslinked polymer absorbed water 40 times by weight as much as the polymer per se on a dry basis. This gel was referred to as G-5.

(II) Comparative Synthesis Example 2

1.5 g of a triiron tetraoxide powder was dispersed in 100 ml of a 3% aqueous solution of agarose in the form of a sol at 45°C. The resulting dispersion was molded into beads of 4 to 5 mm in particle size and cooled to thereby give a gel. This gel was referred to as G-6.

(I) Example 1

Surplus sludge of sewage (sludge concentration: 4000 ppm) was introduced into an aerator (20 l) provided with a diffuser tube at the bottom and air was blown thereinto at a rate of 20 l/min. The water-absorptive resin prepared in each Synthesis Example as described above was added thereto as a carrier in an amount of 100 g (including the magnetic material) on a dry basis. Then an artificial waste water of the following composition was supplied to the aerator at a constant rate. The carrier absorbing water, i.e., the gel in the effluent from the aerator was recovered by using a magnet and returned to the aerator. This treatment was continuously carried out for 30 days at 25°C. Table 1 shows the properties of the water thus treated. Composition of artificial waste water:

oxidized starch: 250 ppm, peptone: 250 ppm,
$KH_2PO_4$: 15 ppm and BOD: 390 ppm.

| | Sample | BOD load ($kg/m^3 \cdot day$) | BOD of treated filtrate (ppm) |
|---|---|---|---|
| Example | G-1 | 4.5 | 5 |
| | G-2 | 6.5 | 11 |
| | G-3 | 5.5 | 7 |
| | G-4 | 4.0 | 9 |
| Comp. Example | G-5 | 1.0 | 390 |
| | G-6 | 1.0 | 390 |

(II) Example 2

The content of a septic tank for human waste and 100 g (including the magnetic material; on a dry basis) of the water-absorptive resin prepared in each Synthetic Example as described above were introduced into a hermetically closed septic tank (20 l) provided with a gas chamber (10 l) at the top and a circulating diffuser tube for blowing the gas from the gas chamber into the bottom. Then an artificial waste water of the following composition was supplied to the septic tank at a constant rate.

The gas chamber was filled with methane gas and the circulation/aeration was continued. The resin

absorbing water, i.e., the gel contained in the effluent from the tank was recovered by using a magnet and returned to the tank. This treatment was continued at 45°C for 30 days. Table 2 shows the properties of the water thus treated.

Composition of artificial waste water:
glucose: 2000 ppm, urea: 200 ppm,
$(NH_4)_4HPO_4$: 20 ppm and BOD: 1400 ppm.

Table 2: Result of anaerobic biological treatment

| | Sample | BOD load ($kg/m^3 \cdot day$) | BOD of treated filtrate (ppm) |
|---|---|---|---|
| Example | G-1 | 6.0 | 230 |
| | G-2 | 7.5 | 210 |
| | G-3 | 7.0 | 190 |
| | G-4 | 6.0 | 280 |
| Comp. Example | G-5 | 1.0 | 1300 |
| | G-6 | 1.0 | 1350 |

**Claims for the Contracting States: DE FR GB NL**

1. A water-absorptive resin for a microbial carrier in a fluidized bed biodisposer, which comprises a magnetic material incorporated in a cationic crosslinked polymer comprising at least 10% by weight of cationic monomer unit(s) of the following formula:

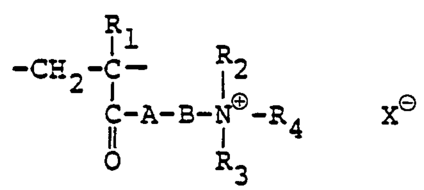

(I)

wherein A represents an oxygen atom or an NH group,
B represents a $C_2H_4$, $C_3H_6$ or $CH_2CH(OH)CH_2$ group,
$R_1$ represents a hydrogen atom or a methyl group,
$R_2$ and $R_3$, which may be the same or different, each independently represent a methyl or ethyl group,
$R_4$ represents a hydrogen atom or a methyl, ethyl, benzyl or 3-chloro-2-hydroxypropyl group, and
$X^-$ represents an anion.

2. A water-absorptive resin according to claim 1, wherein said cationic crosslinked polymer absorbs water at least ten times as much as the polymer per se on a dry basis.

3. A water-absorptive resin according to claim 1 or 2, which serves as a microbial carrier in a fluidized bed in a state of absorbing water.

4. A water-absorptive resin according to claim 1, 2 or 3, wherein said cationic monomer(s) are selected from tertiary amines and quaternary ammonium salts of dialkylaminoalkyl (meth)acrylate and dialkylaminoalkyl (meth)acrylamide.

5. A water-absorptive resin according to any one of the preceding claims, wherein in the cationic monomer unit of formula (I) $R_1$ is a methyl group, $R_2$ and $R_3$ are each a methyl group, $R_4$ is a benzyl group, A is an oxygen atom and B is a $C_2H_4$ group.

6. A water-absorptive resin according to any one of claims 1 to 4, wherein in the cationic monomer unit of formula (I), $R_1$ is a hydrogen atom, $R_2$, $R_3$ and $R_4$ are each a methyl group, A is an oxygen atom and B is a $C_2H_4$ group.

7. A water-absorptive resin according to any one of claims 1 to 4, wherein in the cationic monomer unit of formula (I), $R_1$ is a hydrogen atom, $R_2$, $R_3$ and $R_4$ are each a methyl group, A is an NH group and B is a $C_3H_6$ group.

8. A water-absorptive resin according to any one of the preceding claims, which comprises 90% by weight or less of monomer unit(s) selected from ethylenically unsaturated carboxylic acids, esters thereof and amides thereof.

9. A water-absorptive resin according to claim 8, which comprises 90% by weight or less of (meth)acrylamide monomer unit(s).

10. A water-absorptive resin according to any one of the preceding claims, said resin being obtained by polymerisation of the said monomer or monomers in the presence of 0.01 to 1% by weight, based on the whole monomers, of a crosslinking agent.

11. A water-absorptive resin according to claim 10, wherein said crosslinking agent is N,N-methylenebisacrylamide or N-allylacrylamide, or a mixture thereof.

12. A water-absorptive resin according to claim 10, wherein a polyfunctional compound is used as said crosslinking agent.

13. A water-absorptive resin according to claim 10, wherein the crosslinking agent is selected from epichlorohydrin, diglycidylamine, diglycidyl ether and formaldehyde.

14. A water-absorptive resin according to any one of the preceding claims, wherein the resin is a resin which has been obtained by dispersing an aqueous solution of monomer(s) in an oil and pearl-polymerizing the same.

15. A water-absorptive resin according to any one of the preceding claims, wherein the weight ratio of the magnetic material to the crosslinked polymer on a dry basis, ranges from 1:10 to 10:1.

16. A water-absorptive resin according to any one of the preceding claims, wherein said magnetic material is a ferrite such as magnetite or triiron tetraoxide ($Fe_3O_4$) in the form of particles such as beads, powders or granules.

17. A water-absorptive resin according to any one of claims 1 to 16, wherein said magnetic material is an iron alloy in the form of particles such as beads, powders or granules.

18. A water-absorptive resin according to claim 17, wherein said magnetic material is an iron alloy rendered rustproof by, for example, plating or treatment with silane.

19. A water-absorptive resin according to claim 18, wherein said magnetic material is obtained by absorbing acryloyloxypropyltrimethoxysilane on the surface of the magnetic metal and polymerizing the silane to thereby render said magnetic metal rustproof.

20. A water-absorptive resin according to any one of the preceding claims, wherein said carrier in a state of absorbing water can be recovered by using a magnet.

21. A water-absorptive resin according to any one of the preceding claims, wherein the specific gravity of said carrier in a state of absorbing water is closely similar to that of the water to be treated in an aerator.

22. A water-absorptive resin according to any one of claims 1 to 20, wherein the specific gravity of said carrier in a state of absorbing water is closely similar to that of the water to be treated in a septic tank.

23. A water-absorptive resin according to any one of the preceding claims, wherein said carrier in a state of absorbing water has a particle size of 0.5 to 20 mm.

**Claims for the Contracting State: ES**

1. A process for the preparation of a microbial carrier in a fluidized bed biodisposer which process is embodied in making a water-absorptive resin composite absorb water, wherein the said composite comprises a magnetic material incorporated in a cationic crosslinked polymer comprising at least 10% by weight of cationic monomer unit(s) of the following formula:

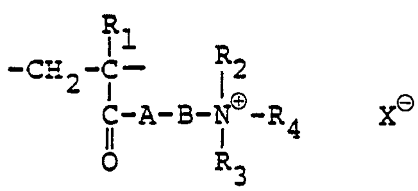

(I)

wherein A represents an oxygen atom or an NH group,
B represents a $C_2H_4$, $C_3H_6$ or $CH_2CH(OH)CH_2$ group,
$R_1$ represents a hydrogen atom or a methyl group,
$R_2$ and $R_3$, which may be the same or different, each independently represent a methyl or ethyl group,
$R_4$ represents a hydrogen atom or a methyl, ethyl, benzyl or 3-chloro-2-hydroxypropyl group, and
$X^-$ represents an anion.

2. A process according to claim 1, wherein said cationic crosslinked polymer absorbs water at least ten times as much as the polymer per se on a dry basis.

3. A process according to claim 1 or 2, wherein said cationic monomer(s) are selected from tertiary amines and quaternary ammonium salts of dialkylaminoalkyl (meth)acrylate and dialkylaminoalkyl (meth)acrylamide.

4. A process according to any one of the preceding claims, wherein in the cationic monomer unit of formula (I), $R_1$ is a methyl group, $R_2$ and $R_3$ are each a methyl group, $R_4$ is a benzyl group, A is an oxygen atom and B is a $C_2H_4$ group.

5. A process according to any one of claims 1 to 3, wherein in the cationic monomer unit of formula (I), $R_1$ is a hydrogen atom, $R_2$, $R_3$ and $R_4$ are each a methyl group, A is an oxygen atom and B is a $C_2H_4$ group.

6. A process according to any one of claims 1 to 3, wherein in the cationic monomer unit of formula (I), $R_1$ is a hydrogen atom, $R_2$, $R_3$ and $R_4$ are each a methyl group, A is an NH group and B is a $C_3H_6$ group.

7. A process according to any one of the preceding claims, wherein the water-absorptive resin comprises 90% by weight or less of monomer unit(s) selected from ethylenically unsaturated carboxylic acids, esters thereof and amides thereof.

8. A process according to claim 7, wherein the water-absorptive resin comprises 90% by weight or less of (meth)acrylamide monomer unit(s).

9. A process according to any one of the preceding claims, wherein the water-absorptive resin has been obtained by polymerisation of the said monomer or monomers in the presence of 0.01 to 1% by weight, based on the whole monomers, of a crosslinking agent.

10. A process according to claim 9, wherein said crosslinking agent is N,N-methylenebisacrylamide or N-allylacrylamide, or a mixture thereof.

11. A process according to claim 9, wherein a polyfunctional compound is used as said crosslinking agent.

12. A process according to claim 9, wherein the crosslinking agent is selected from epichlorohydrin, diglycidylamine, diglycidyl ether and formaldehyde.

13. A process according to any one of the preceding claims, wherein the water-absorptive resin is a resin which has been obtained by dispersing an aqueous solution of monomer(s) in an oil and pearl-polymerizing the same.

14. A process according to any one of the preceding claims, wherein the weight ratio of the magnetic material to the crosslinked polymer on a dry basis, ranges from 1:10 to 10:1.

15. A process according to any one of the preceding claims, wherein said magnetic material is a ferrite such as magnetite or triiron tetraoxide ($Fe_3O_4$) in the form of particles such as beads, powders or granules.

16. A process according to any one of claims 1 to 15, wherein said magnetic material is an iron alloy in the form of particles such as beads, powders or granules.

17. A process according to claim 16, wherein said magnetic material is an iron alloy rendered rustproof by, for example, plating or treatment with silane.

18. A process according to claim 17, wherein said magnetic material is obtained by absorbing acryloyloxypropyltrimethoxysilane on the surface of the magnetic metal and polymerizing the silane to thereby render said magnetic metal rustproof.

19. A process according to any one of the preceding claims, wherein said carrier in a state of absorbing water can be recovered by using a magnet.

20. A process according to any one of the preceding claims, wherein the specific gravity of said carrier in a state of absorbing water is closely similar to that of the water to be treated in an aerator.

21. A process according to any one of claims 1 to 19, wherein the specific gravity of said carrier in a state of absorbing water is closely similar to that of the water to be treated in a septic tank.

22. A process according to any one of the preceding claims, wherein said carrier in a state of absorbing water has a particle size of 0.5 to 20 mm.

**Patentansprüche für die Vertragsstaaten: DE FR GB NL**

1. Wasserabsorbierendes Kunstharz für einen mikrobiologischen Träger in einem Wirbelbett-Bioreaktor enthaltend ein magnetisches Material in einem kationischen vernetzten Polymeren mit mindestens 10 Gew.-% an kationischen Monomereneinheit(en) der folgenden Formel (I)

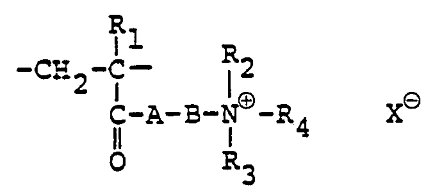

(I)

worin

A ein Sauerstoffatom oder eine NH-Gruppe ist,

B eine $C_2H_4$—, $C_3H_6$ oder $CH_2CH(OH)CH_2$-Gruppe ist,

$R_1$ ein Wasserstoffatom oder eine Methylgruppe ist,

$R_2$ und $R_3$, gleich oder unterschiedlich, jeweils unabhängig voneinander eine Methyl- oder Ethylgruppe bedeuten,

$R_4$ ein Wasserstoffatom oder eine Methyl-, Ethyl-, Benzyl- oder 3-Chlor-2-hydroxypropylgruppe darstellt und

$X^-$ ein Anion ist.

2. Wasserabsorbierendes Kunstharz nach Anspruch 1, in dem das kationische vernetzte Polymere mindestens das Zehnfache der Menge des Polymeren an Wasser absorbiert, bezogen auf die Trockensubstanz.

3. Wasserabsorbierendes Kunstharz nach Anspruch 1 oder 2, zur Verwendung als mikrobiologischer Träger in wasserabsorbierender Form in einem Wirbelbett.

4. Wasserabsorbierendes Kunstharz nach einem der Ansprüche 1 bis 3, in dem das kationische Monomere oder die kationischen Monomeren aus tertiären Aminen und quartären Ammoniumsalzen von Dialkylaminoalkyl (meth)acrylat und Dialkylaminoalkyl(meth)acrylamid ausgewählt ist (sind).

5. Wasserabsorbierendes Kunstharz nach einem der vorhergehenden Ansprüche, wobei in der kationischen Monomereneinheit der Formel (I) $R_1$ eine Methylgruppe, $R_2$ und $R_3$ jeweils eine Methylgruppe, $R_4$ eine Benzylgruppe, A ein Wasserstoffatom und B eine $C_2H_4$-Gruppe bedeuten.

6. Wasserabsorbierendes Kunstharz nach einem der Ansprüche 1 bis 4, wobei in der kationischen Monomereneinheit der Formel (I) $R_1$ ein Wasserstoffatom, $R_2$, $R_3$ und $R_4$ jeweils eine Methylgruppe bedeuten, A ein Sauerstoffatom und B eine $C_2H_4$-Gruppe bedeuten.

7. Wasserabsorbierendes Kunstharz nach einem der Ansprüche 1 bis 4, wobei in der kationischen Monomereneinheit der Formel (I) $R_1$ ein Wasserstoffatom, $R_2$, $R_3$ und $R_4$ jeweils eine Methylgruppe bedeuten, A eine HN-Gruppe und B eine $C_3H_6$-Gruppe darstellen.

8. Wasserabsorbierendes Kunstharz nach einem der vorhergehenden Ansprüche, welches 90% oder weniger an Monomereneinheit(en) umfasst, die ausgewählt sind aus ethylenisch ungesättigten Carbonsäuren, sowie deren Estern und Amiden.

9. Wasserabsorbierendes Kunstharz nach Anspruch 8, welches 90% oder weniger an (Meth)acrylamid-Monomereneinheiten enthält.

10. Wasserabsorbierendes Kunstharz nach einem der vorhergehenden Ansprüche, welches erhalten wird durch Polymerisation des order der genannten Monomeren in Gegenwert von 0.1 bis 1 Gew.-% eines Vernetzungsmittels bezogen auf das Gesamtgewicht an Monomeren.

11. Wasserabsorbierendes Kunstharz nach Anspruch 10, wobei das Vernetzungsmittel, N,N-Methylenbisacrylamid oder N-Allylacrylamid oder eine Mischung derselben ist.

12. Wasserabsorbierendes Kunstharz nach Anspruch 10, worin eine polyfunktionelle Verbindung als Netzmittel verwendet ist.

13. Wasserabsorbierendes Kunstharz nach Anspruch 10, wobei das Netzmittel ausgewählt ist aus Epichlorhydrin, Diglycidylamin, Diglycidylether und Formaldehyd.

14. Wasserabsorbierendes Kunstharz nach einem der vorhergehenden Ansprüche, wobei das Kunstharz erhalten worden ist durch Dispergieren einer wässrigen Lösung von Monomer(en) in einem Öl und Perlpolymerisation des- bzw. derselben.

15. Wasserabsorbierendes Kunstharz nach einem der vorhergehenden Ansprüche, worin das Gewichtsverhältnis des magnetischen Materials zu dem vernetzten Polymeren 1:10 bis 10:1, bezogen auf die Trockensubstanz, beträgt.

16. Wasserabsorbierendes Kunstharz nach einem der vorhergehenden Ansprüche, wobei das magnetische Material ein Ferrit, zum Beispiel Magnetit oder Eisentetraoxid ($Fe_3O_4$) in Form von Teilchen, beispielsweise Perlen, Pulver oder Granulat ist.

17. Wasserabsorbierendes Kunstharz nach einem der Ansprüche 1 bis 16, worin das magnetische Material als Eisenlegierung in Form von Teilchen, beispielsweise Perlen, Pulver oder Granulat, vorliegt.

18. Wasserabsorbierendes Kunstharz nach Anspruch 17, wobei das magnetische Material eine Eisenlegierung ist, die beispielsweise durch Plattieren oder durch Behandlung mit Silan korrosionsbeständig gemacht worden ist.

19. Wasserabsorbierendes Kunstharz nach Anspruch 18, wobei das magnetische Material erhalten wird durch Absorption von Acryloyloxypropyltrimethoxysilan an die Oberfläche des magnetischen Metalls und Polymerisation des Silans, um so das magnetische Metall korrosionsfest zu machen.

20. Wasserabsorbierendes Kunstharz nach einem der vorhergehenden Ansprüche, wobei der Träger in einem wasserabsorbierenden Zustand durch Einsatz eines Magneten wiedergewonnen werden kann.

21. Wasserabsorbierendes Kunstharz nach einem der vorhergehenden Ansprüche, wobei das spezifische Gewicht des Trägers im Wasserabsorbierenden Zustand eng verwandt ist mit dem von in einem Belüfter zu behandelnden Wasser ist.

22. Wasserabsorbierendes Kunstharz nach einem der Ansprüche 1 bis 20, wobei das spezifische Gewicht des Trägers in wasserabsorbierender Form dem von Wasser sehr ähnlich ist, das in einem Faulbehälter zu behandeln ist.

23. Wasserabsorbierendes Kunstharz nach einem der vorhergehenden Ansprüche, in dem der Träger in Wasser absorbierender Form eine Teilchengrösse von 0,5 bis 20 mm hat.

**Patentansprüche für den Vertragsstaat: ES**

1. Verfahren zur Herstellung eines mikrobiologischen Trägers in einem Wirbelbett-Bioreaktor, in dem eine wasserabsorbierende Kunstharz-Zusammensetzung dazu veranlasst wird, Wasser zu absorbieren, wobei die Zusammensetzung ein magnetisches Material in einem kationischen vernetzten Polymeren

umfasst, das mindestens 10 Gew.-% an kationischen monomereneinheit(en) der folgenden Formel umfasst,

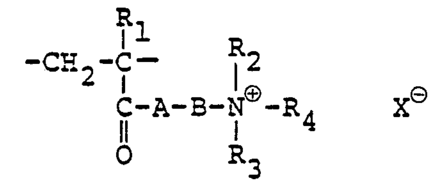 (I)

worin

A ein Sauerstoffatom oder eine NH-Gruppe ist,

$R_2$ und $R_3$, gleich oder unterschiedlich, jeweils unabhängig voneinander eine Methyl- oder Ethylgruppe bedeuten,

$R_4$ ein Wasserstoffatom oder eine Methyl-, Ethyl-, Benzyl- oder 3-Chlor-2-hydroxypropylgruppe darstellt und

$X^-$ ein Anion ist.

2. Wasserabsorbierendes Kunstharz nach Anspruch 1, in dem das kationische vernetzte Polymere mindestens das Zehnfache der Menge des Polymeren an Wasser absorbiert, bezogen auf die Trockensubstanz.

3. Verfahren nach Anspruch 1 oder 2, wobei das (die) kationische(n) Monomere(n) ausgewählt werden aus tertiären Aminen und quartären Ammoniumsalzen von Dialkylaminoalkyl(meth)acrylamid.

4. Verfahren nach einem der vorhergehenden Ansprüche, wobei in die kationische Monomereneinheit der Formel (I) eine Methylgruppe, $R_4$ eine Benzylgruppe, A ein Sauerstoffatom und B eine $C_2H_4$-Gruppe bedeuten.

5. Verfahren nach einem der Ansprüche 1 bis 3, wobei in der kationischen Monomereneinheit der Formel (I) $R_1$ ein Wasserstoffatom, $R_2$, $R_3$ und $R_4$ jeweils eine Methylgruppe darstellen, A ein Sauerstoffatom und B eine $C_2H_4$-Gruppe bedeuten.

6. Verfahren nach einem der Ansprüche 1 bis 3, wobei in der kationischen Monomereneinheit der Formel (I) $R_1$ ein Wasserstoffatom, $R_2$, $R_3$ und $R_4$ jeweils eine Methylgruppe, A eine NH-Gruppe und B eine $C_3H_6$-Gruppe darstellen.

7. Verfahren nach einem der vorhergehenden Ansprüche, wobei das wasserabsorbierende Kunstharz 90 Gew.-% oder weniger an Monomereneinheiten umfasst, die ausgewählt sind aus ethylenisch ungesättigten Carbonsäuren, deren Estern und Amiden.

8. Verfahren nach Anspruch 7, wobei das wasserabsorbierende Kunstharz 90 Gew.-% oder weniger an (Meth)acrylamidmonomereneinheit(en) umfasst.

9. Verfahren nach einem der vorhergehenden Ansprüche, worin das wasserabsorbierende Harz erhalten wurde durch Polymerisation des (der) genannten Monomeren in Gegenwert von 0.01 bis 1 Gew.-% des Gesamtmonomerengehalts an einem Vernetzungsmittel.

10. Verfahren nach Anspruch 9, wobei das Vernetzungsmittel, N,N-Methylenbisacrylamid oder N-Allylacrylamid oder ein Gemisch der beiden ist.

11. Verfahren nach Anspruch 9, wobei eine polyfunktionelle Verbindung als Vernetzungsmittel verwendet wird.

12. Verfahren nach Anspruch 9, wobei das Vernetzungsmittel ausgewählt wird aus Epichlorhydrin, Diglycidylamin, Diglycidyläther und Formaldehyd.

13. Verfahren nach einem der vorhergehenden Ansprüche, worin das wasserabsorbierende Kunstharz erhalten worden ist durch Dispergieren einer wässrigen Lösung von Monomerem(en) in einem Öl und Perlpolymerisation derselben.

14. Verfahren nach einem der vorhergehenden Ansprüche, wobei das Gewichtsverhältnis von magnetischen Material zu dem vernetzten Polymeren, bezogen auf die Trockensubstanz, von 1:10 bis 10:1 beträgt.

15. Verfahren nach einem der vorhergehenden Ansprüche, wobei das magnetische Material ein Ferrit ist, wie zum Beispiel Magnetit oder Eisentetraoxyd ($Fe_3O_4$) in Form von Teilchen, wie zum Beispiel Perlen, Pulver oder Körnern vorliegt.

16. Verfahren nach einem der Ansprüche 1 bis 15, wobei das magnetische Material eine Eisenlegierung ist, die in Form von Teilchen, beispielsweise Perlen, Pulver oder Granulat vorliegt.

17. Verfahren nach Anspruch 16, wobei das magnetische Material eine beispielsweise durch Plattieren oder Behandlung mit Silan korrosionsbeständig gemacht Eisenlegierung ist.

18. Verfahren nach Anspruch 17, in dem das magnetische Material durch Absorption von Acryloyloxypropyltrimethoxysilan an die Oberfläche des magnetischen Metalls und Polymerisation des Silans erhalten wird, wodurch das magnetische Metall korrosionsfest wird.

19. Verfahren nach einem der vorhergehenden Ansprüche, wobei der Träger in wasserabsorbierenden Zustand durch Anwendung eines Magneten zurückgewonnen werden kann.

20. Verfahren nach einem der vorhergehenden Ansprüche, wobei das spezifische Gewicht des Trägers in Wasserabsorbierender Form in wesentlichen dem von Wasser gleicht, das in einer Belüftungsvorrichtung zu behandeln ist.

21. Verfahren nach einem der Ansprüche 1 bis 19, wobei das spezifische Gewicht des Trägers in wasserabsorbierender Form im wesentlich dem von Wasser gleicht, das in einem Faulbehälter zu behandeln ist.

22. Verfahren nach einem der vorhergehenden Ansprüche, wobei der Träger in wasserabsorbierender Form eine wasserabsorbierender Form eine Teilchengrösse von 0,5 bis 20 mm aufweist.

**Revendications pour les Etats contractants: DE FR GB NL**

1. Résine absorbant l'eau destinée à un support microbien dans un épurateur biologique à lit fluidisé, qui comprend une matière magnétique incorporée à un polymère cationique réticulé renfermant au moins 10% en poids d'un ou plusieurs motifs monomériques cationiques répondant à la formule suivante:

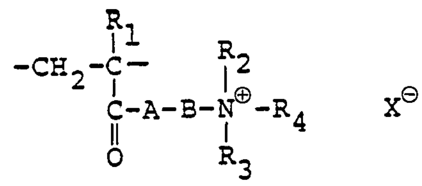

(I)

dans laquelle A représente un atome d'oxygène ou un groupe NH,

B représente un groupe $C_2H_4$, $C_3H_6$ ou $CH_2CH(OH)CH_2$,

$R_1$ représente un atome d'hydrogène ou un groupe méthyle,

$R_2$ et $R_3$, qui peuvent être identiques ou différents, représentent chacun indépendamment un groupe méthyle ou éthyle,

$R_4$ représente un atome d'hydrogène ou un groupe méthyle, éthyle, benzyle ou 3-chloro-2-hydroxypropyle,

et $X^-$ représente un anion.

2. Résine absorbant l'eau suivant la revendication 1, dans laquelle le polymère cationique réticulé absorbe une quantité d'eau égale à au moins dix fois la quantité du polymère proprement dit, sur base sèche.

3. Résine absorbant l'eau suivant la revendication 1 ou 2, qui sert de support microbien dans un lit fluidisé dans une phase d'absorption d'eau.

4. Résine absorbant l'eau suivant la revendication 1, 2 ou 3, dans laquelle le ou les monomères cationiques sont choisis entre des sels d'amines tertiaires et d'ammonium quaternaire de (méth)acrylate de dialkylaminoalkyle et de dialkylaminoalkyl-(méth)acrylamide.

5. Résine absorbant l'eau suivant l'une quelconque des revendications précédentes, dans laquelle, dans la motif monomérique cationique de formule (I), $R_1$ représente un groupe méthyle, $R_2$ et $R_3$ représentent chacun un groupe méthyle, $R_4$ représente un groupe benzyle, A représente un atome d'oxygène et B représente un groupe $C_2H_4$.

6. Résine absorbant l'eau suivant l'une quelconque des revendications 1 à 4, dans laquelle, dans le un atome d'hydrogène, $R_2$, $R_3$ et $R_4$ représentent chacun un groupe méthyle, A représente un atome d'oxygéne et B représente un groupe $C_2H_4$.

7. Résine absorbant l'eau suivant l'une quelconque des revendications 1 à 4, dans laquelle, dans le motif monomérique cationique de formule (I), $R_1$ represénte un atome d'hydrogène, $R_2$, $R_3$ et $R_4$ représentent chacun un groupe méthyle, A représente un groupe NH et B représente un groupe $C_3H_6$.

8. Résine absorbant l'eau suivant l'une quelconque des revendications précédentes, qui comprend une quantité égale ou inférieure à 90% en poids d'un ou plusieurs motifs monomériques choisis entre des acides carboxyliques à insaturation éthylénique, leurs esters et leurs amides.

9. Résine absorbant l'eau suivant la revendication 8, qui comprend une quantité égale ou inférieure à 90% en poids d'un ou plusieurs motifs monomériques du type (méth)acrylamide.

10. Résine absorbant l'eau suivant l'une quelconque des revendications précédentes, ladite résine étant obtenue par polymérisation du ou des monomères en présence de 0,01 à 1% en poids, sur la base des monomères totaux, d'un agent de réticulation.

11. Résine absorbant l'eau suivant la revendications 10, dans laquelle l'agent de réticulation est N,N-méthylène-bis-acrylamide ou le N-allylacrylamide, ou un de leurs mélanges.

12. Résine absorbant l'eau suivant la revendications 10, dans laquelle un composé polyfonctionnel est utilisé comme agent de réticulation.

13. Résine absorbant l'eau suivant la revendications 10, dans laquelle l'agent de réticulation est choisi entre l'épichlorhydrine, la diglycidylamine, l'éther de diglycidyle et le formaldéhyde.

14. Résine absorbant l'eau suivant l'une quelconque des revendications précédentes, dans laquelle la résine est une résine qui a été obtenue en dispersant une solution aqueuse d'un ou plusieurs monomères dans une huile et en polymérisant en perles ladite dispersion.

15. Résine absorbant l'eau suivant l'une quelconque des revendications précédentes, dans laquelle le rapport pondéral de la matière magnétique au polymère réticulé, sur base sèche, va de 1:10 à 10:1.

16. Résine absorbant l'eau suivant l'une quelconque des revendications précédentes, dans laquelle la

matière magnétique est une ferrite telle que la magnétite ou tétraoxyde ferrique ($Fe_3O_4$) sous forme de particules telles que des perles, des poudres ou des granules.

17. Résine absorbant l'eau suivant l'une quelconque des revendications 1 à 16, dans laquelle la matière magnétique est un alliage de fer sous forme de particules telles que des perles, des poudres ou des granules.

18. Résine absorbant l'eau suivant la revendications 17, dans laquelle la matière magnétique est un alliage de fer rendu résistant à la rouille par, par exemple, placage ou traitement avec un silane.

19. Résine absorbant l'eau suivant la revendications 18, dans laquelle la matière magnétique est obtenue par absorption d'un acryloyloxypropyltriméthoxysilane sur la surface du métal magnétique et polymérisation du silane pour rendre ainsi ledit métal magnétique résistant à la rouille.

20. Résine absorbant l'eau suivant l'une quelconque des revendications précédentes, dans laquelle le support, en phase d'absorption d'eau, peut être séparé en utilisant un aimant.

21. Résine absorbant l'eau suivant l'une quelconque des revendications précédentes, dans laquelle la densité du support, en phase d'absorption d'eau, est très proche de celle de l'eau à traiter dans une aérateur.

22. Résine absorbant l'eau suivant l'une quelconque des revendications 1 à 20, dans laquelle la densité du support, en phase d'absorption d'eau, est très proche de celle de l'eau à traiter dans une fosse septique.

23. Résine absorbant l'eau suivant l'une quelconque des revendications précédentes, dans laquelle le support, en phase d'absorption d'eau, possède un diamètre de particules de 0,5 à 20 mm.

**Revendications pour l'Etat contractant: ES**

1. Procédé de préparation d'un support microbien dans un épurateur biologique à lit fluidisé, procédé qui consiste à préparer une résine composite absorbant l'eau, ladite résine composite comprenant une matière magnétique incorporée à un polymère cationique réticulé renfermant au moins 10% en poids d'un ou plusieurs motifs monomériques cationiques répondant à la formule suivante:

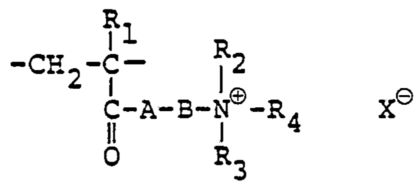

(I)

dans laquelle A représente un atome d'oxygène ou un groupe NH,

B représente un groupe $C_2H_4$, $C_3H_6$ ou $CH_2CH(OH)CH_2$,

$R_1$ représente un atome d'hydrogène ou un groupe méthyle,

$R_2$ et $R_3$, qui peuvent être identiques ou différents, représentent chacun indépendamment un groupe méthyle ou éthyle,

$R_4$ représente un atome d'hydrogène ou un groupe méthyle, éthyle, benzyle ou 3-chloro-2-hydroxypropyle,

et $X^-$ représente un anion.

2. Procédé suivant la revendication 1, dans laquelle le polymère cationique réticulé absorbe une quantité d'eau égale à au moins dix fois la quantité du polymère proprement dit, sur base sèche.

3. Procédé suivant la revendication 1, ou 2, dans lequel le ou les monomères cationiques sont choisis entre des sels d'amines tertiaires et d'ammonium quaternaire de (méth)acrylate de dialkylaminoalkyle et de dialkylaminoalkyl-(méth)acrylamide.

4. Procédé suivant l'une quelconque des revendications précédentes, dans lequel, dans le motif monomérique cationique de formule (I), $R_1$ représente un groupe méthyle, $R_2$ et $R_3$ représentent chacun un groupe méthyle, $R_4$ représente un groupe benzyle, A représente un atome d'oxygène et B représente un groupe $C_2H_4$.

5. Procédé suivant l'une quelconque des revendications 1 à 3, dans lequel, dans le motif monomérique cationique de formule I, $R_1$ représente un atome d'hydrogène, $R_2$, $R_3$ et $R_4$ représentent chacun un groupe méthyle, A représente un atome d'oxygéne et B représente un groupe $C_2H_4$.

6. Procédé suivant l'une quelconque des revendications 1 à 3, dans lequel, dans le motif monomérique cationique de formule (I), $R_1$ represénte un atome d'hydrogène, $R_2$, $R_3$ et $R_4$ représentent chacun un groupe méthyle, A représente un groupe NH et B représente un groupe $C_3H_6$.

7. Procédé suivant l'une quelconque des revendications précédentes, dans lequel la résine absorbant l'eau comprend une quantité égale ou inférieure à 90% en poids d'un ou plusieurs motifs monomériques choisis entre des acides carboxyliques à insaturation éthylénique, leurs esters et leurs amides.

8. Procédé suivant la revendication 7, dans lequel la résine absorbant l'eau comprend une quantité égale ou inférieure à 90% en poids d'un ou plusieurs motifs monomériques (méth)acrylamide.

9. Procédé suivant l'une quelconque des revendications précédentes, dans lequel la résine absorbant l'eau a été obtenue par polymérisation du ou des monomères en présence d'une quantité de 0,01 à 1% en poids, sur la base des monomères totaux, d'un agent de réticulation.

10. Procédé suivant la revendications 9, dans lequel l'agent de réticulation est le N,N-méthylène-bis-acrylamide ou le N-allylacrylamide, ou un de leurs mélanges.

11. Procédé suivant la revendications 9, dans lequel un composé polyfonctionnel est utilisé comme agent de réticulation.

12. Procédé suivant la revendications 9, dans lequel l'agent de réticulation est choisi entre l'épichlorhydrine, la diglycidylamine, l'éther de diglycidyle et le formaldéhyde.

13. Procédé suivant l'une quelconque des revendications précédentes, dans lequel la résine absorbant l'eau est une résine qui a été obtenue en dispersant une solution aqueuse d'un ou plusieurs monomères dans une huile et en polymérisant en perles ladite dispersion.

14. Procédé suivant l'une quelconque des revendications précédentes, dans lequel le rapport pondéral de la matière magnétique au polymère réticulé, sur base sèche, est compris dans l'intervalle de 1:10 à 10:1.

15. Procédé suivant l'une quelconque des revendications précédentes, dans lequel la matière magnétique est une ferrite telle que la magnétite ou tétraoxyde ferrique ($Fe_3O_4$) sous forme de particules telles que des perles, des poudres ou des granules.

16. Procédé suivant l'une quelconque des revendications 1 à 15, dans lequel la matière magnétique est un alliage de fer sous forme de particules telles que des perles, des poudres ou des granules.

17. Procédé suivant la revendications 16, dans lequel la matière magnétique est un alliage de fer rendu résistant à la rouille, par exemple par placage ou traitement avec un silane.

18. Procédé suivant la revendication 17, dans lequel la matière magnétique est obtenue par absorption d'acryloyloxypropyltriméthoxysilane sur la surface de la matière magnétique et polymérisation du silane pour rendre ainsi ledit métal magnétique résistant à la rouille.

19. Procédé suivant l'une quelconque des revendications précédentes, dans lequel le support, en phase d'absorption d'eau, peut être séparé en utilisant un aimant.

20. Procédé suivant l'une quelconque des revendications précédentes, dans lequel la densité du support, en phase d'absorption d'eau, est très proche de celle de l'eau à traiter dans une aérateur.

21. Procédé suivant l'une quelconque des revendications 1 à 19, dans lequel la densité du support, en phase d'absorption d'eau, est très proche de celle de l'eau à traiter dans une fosse septique.

22. Procédé suivant l'une quelconque des revendications précédentes, dans lequel le support, en phase d'absorption d'eau, possède un diamètre de particules de 0,5 à 20 mm.